# NEW EUROPEAN PATENT SPECIFICATION

(11) **EP 1 862 160 B2**
(45) Date of publication and mention of the opposition decision: **20.04.2016**
(45) Mention of the grant of the patent: 27.02.2013
(21) Application number: 07010175.3
(22) Date of filing: 22.05.2007
(51) Int. Cl.: A61K 8/46, A61K 8/73, A61K 8/891, A61Q 5/02

(54) **Aqueous hair cleansing agent**
Wässriges Haarwaschmittel
Agent aqueux de nettoyage de cheveux

(30) Priority: 22.05.2006 JP 2006141454
(43) Date of publication of application: 05.12.2007
(73) Proprietor: Kao Corporation, Chuo-ku Tokyo 103-8210 (JP)
(72) Inventor: Terada, Eiji, Tokyo 131-8501 (JP)
(74) Representative: Hoffmann Eitle

(56) References cited:
- EP-A1- 0 468 721
- EP-A1- 1 226 814
- EP-A1- 1 226 814
- WO-A1-2006/052580
- JP-A- 2003 327 603
- JP-A- 2006 062 979
- US-A1- 2006 099 167

## Description

### FIELD OF THE INVENTION

The present invention relates to an aqueous hair cleansing agent.

### BACKGROUND OF THE INVENTION

Dimethylpolysiloxanes have been conventionally used for hair cleansing agents for the purpose of imparting conditioning effects to the hair. Dimethylpolysiloxanes are effective for improving the smoothness of the hair during foaming, but they do not remain on the hair during rinsing so that it is difficult to impart smoothness to the hair by using dimethylpolysiloxanes alone. For efficienty leaving dimethylpolysiloxanes on the hair, proposed is a process of using cationized guar gum and a dimethylpolysi oxane having an average particle size less than 2 µm in combination (JP-A-4-234309). The dimethylpolysiloxane is however dispersed in water relatively stably because of its average particle size of as small as 2 µm so that it does not remain on the hair sufficiently. In addition, cationized guar gum causes entanglement of the hair during rinsing because it does not sufficiently provide smoothness to the hair during rinsing.

In order to improve conditioning effects, use of a cationized tara gum obtained by cationizing a polysaccharide available from the endosperm of the seeds of tara (*Caesal pinia Spinosa*) which is a leguminous plant is proposed (JP-A-2004-203801). Use of a cationized tara gum with cationic guar gum in combination in a hair cleansing agent is also proposed (JP-A-2005-272658). These prior arts disclose some examples using a dimethylpolysiloxane in the form of an aqueous emulsion, but the dimethylpolysiloxane therein has an average particle size less than 1 µm so that the above-described problem, that is, entanglement of the hair during rinsing due to insufficient remaining of it on the hair has not been overcome yet.

### SUMMARY OF THE INVENTION

The present invention provides an aqueous hair cleansing agent comprising the following components (A), (B) and (C):
(A) an anionic surfactant,
(B) a cationized tara gum, and
(C) a dimethylpolysiloxane present as dispersed particles having an average particle size of from 2 to 100 µm, and
(D) a glyceryl ether selected from the group consisting of monoalkyl glyceryl ethers having a C₄₋₁₀ alkyl group and monoalkenyl glyceryl ethers having a C₄₋₁₀ alkenyl group.

### BRIEF DESCRIPTION OF THE DRAWINGS

FIG. 1 is an overal view of an apparatus employed for the evaluation of foaming speed; and
FIG. 2(a) is a top view of a disk to which hairs are to be implanted and FIG. 2(b) is a cross-sectional view of a disk to which hairs have been implanted.

- 1:: Hair
- 10:: Container
- 11:: Disk
- 20:: Lid
- 21:: First protrusion
- 22:: Second protrusion
- 23:: Foam inducing wall
- 24:: Hair-entrapping-prevention pin
- 25:: Inlet
- 30:: Motor
- 40:: Control unit
- 41:: Support
- 50:: Metering instrument
- 51:: Water inlet
- 60:: Torque detector
- P:: Implant nole

### DETAILED DESCRIPTION OF THE INVENTION

The present invention relates to an aqueous hair cleansing agent which provides, even if used for the hair damaged by chemical treatment, rapid foaming and smooth feeling of foam during shampooing, provides smooth feel to the hair during rinsing, provides the finished hair with luster and manageability, and has excellent storage stability.

The present inventor has found out an aqueous hair cleansing agent which is excellent in the performance and feel of foams, and finish, even if it is applied to the damaged hair, and also is excellent in storage stability by using, in combination, an anionic surfactant, a cationized tara gum and a dimethylpolysiloxane having a specific particle size.

As the anionic surfactant of Component (A), sulfate-, sulfonate- and carboxylate-based surfactants are usable. Specific examples include alkyl sulfates, alkenyl sulfates, polyoxyalkylene alkyl ether sulfates, polyoxyalkylene alkenyl ether sulfates, alkyl sulfosuccinates, polyoxyalkylene alkyl sulfosuccinates, alkane sulfonates, higher fatty acid salts, and alkyl ether carboxylates. Of these, polyoxyalkylene alkyl ether sulfates, polyoxyalkylene alkenyl ether sulfates, alkyl sulfates, and alkenyl sulfates are preferred, with those represented by the following formula (1) or (2) being more preferred:

R¹O(CH₂CH₂O)ₘSO₃M (1)

R¹OSO₃M (2)

wherein, R¹ represents a C₁₀₋₁₈ alkyl or alkenyl group, M represents an alkali metal, alkaline earth metal, ammonium, a cation derived from alkanolamine or basic amino acid, and m stands for an average number by weight of from 1 to 5.

Of these, polyoxyethylene alkyl ether sulfates of the formula (1) in which R¹ represents a C₁₂₋₁₄ alkyl group, m stands for a number of 1 on weight average and M represents ammonium or sodium are preferred because they can satisfy both speedy foaming and good feel of foams.

As Component (A), the above-described surfactants may be used either singly or in combination two or more thereof. The content of it in the aqueous hair cleansing agent of the present invention preferably ranges from 1 to 30 weight%, more preferably from 5 to 25 weight%, even more preferably from 8 to 20 weight% from the viewpoints of foaming property, liquid state during use and cleansing property.

Component (B) of the present invention is a cationized tara gum. Tara gum, which is an origin of the cationized tara gum, is obtained from the endosperm of the seeds of tara (*Caesalpinia spinosa*) belonging to leguminous plants and is a galactomannan polysaccharide having, as a main chain, mannose and, as a side chain linked to the main chain, galactose at a ratio of 3:1.

In the cationized tara gum to be used in the present invention, some hydroxyl groups contained in the galactomannan structure in the tara gum have been substituted with a quaternary nirogen-containing group. More in detail, it can be prepared by reacting tara gum with a compound having a quarternary nitrogen-containing group such as glycidyl trialkylammonium salt or 3-halogeno-2-hydroxypropyltrialkylammonium salt. This reaction is effected in the presence of an alkali in a proper solvent, preferably a hydrous alcohol. Such introduction of a quaternary nitrogen-containing group can be performed in accordance with the conventionally known manner.

Although no particular limitation is imposed on the cationization degree of a cationized tara gum, that is the average number of moles of the quaternary nitrogen-containing group to be added permonosaccharide unit, it is preferably from 0.1 to 0.5. A cationized tara gum having a cationization degree less than 0.1 does not exhibit sufficient conditioning effects because an adsorption amount of it to the hair is insufficient. A cationized tara gum having a cationization degree exceeding 0.5, on the other hand, causes a sticky feel during use and moreover worsens foamability. Examples of commercially available products include "Catinal CTR-100" and "Catinal CTR-200" (each, product of Toho Chemical Industry).

As Component (B), these cationized tara gums can be used either singly or in combination of two or more. The content of Component (B) in the aqueous hair cleansing agent of the present invention is preferalby from 0.01 to 10 weight%, more preferably from 0.02 to 5 weight%, even more preferably from 0.03 to 2 weight% from the standpoints of decreasing friction between individual hairs during rinsing.

Component (C) is a dimethylpolysiloxane present as dispersed particles having an average particle size of from 2 to 100 µm. Examples of the dimethylpolysiloxane include those represented by the following formula (3):

R²(CH₃)₂SiO-[(CH₃)₂SiO]ₙ-Si(CH₃)₂R² (3)

wherein, R² each independently represents a methyl or hydroxyl group and n stands for from 1 to 20,000.

The dimethylpolysiloxane as Comporient (C) has a kinematic viscosity at 25°C of from 10 to 30,000,000 mm²/s, more preferably from 1,000 to 20,000,000 mm²/s, even more preferably from 10,000 to 10,000,000 mm²/s from the standpoints of smoothness during foaming and rinsing, and nonsticky feel after drying. Examples of the commercially available dimethylpolysiloxane include "KF-96A-5cs", "KF-96A-10cs", "KF-96A-100cs", "KF-96A-1000cs", "KF-96A-5000cs", "KF-96H-10000cs", "KF-96H-50000cs", "KF-96H-100000cs", and "KF-96H-1000000cs" (product of Shin-Etsu Silicone, respectively). These dimethylpolysiloxanes may be used either singly or in combination of two or more. In addition, those commercially available as a mixture with cyclic silicone in the liquid form such as octamethylcyclotetrasiloxane, decamethylcyclopentasiloxane and dodecamethylcyclohexasiloxane are also usable.

The dimethylpolysiloxane as Component (C) is used preferably in the form of an aqueous emulsion formed mechanically in advance. When the aqueous emulsion is prepared, addition of an least one emulsifier is preferred in order to stabilize the emulsion.

Examples of the emulsifier usable for the preparation include nonionic surfactants such as polyoxyethylene alkyl ethers, polyoxyethylene alkylphenyl ethers, polyoxyethylene sorbitan fatty acid esters, polyoxyethylene glyceryl fatty acid esters, polyoxyethylene hydrogenated castor oil, polyethylene glycol fatty acid esters, polyglycerin fatty acid esters, sucrose fatty acid esters, and polyether modified silicones; cationic surfactants such as alkyltrimethylammonium chlorides, dialkyldimethylammonium chlorides, alkyldimethylamine salts, alkoxydimethylamine salts, and alkylamidodimethylamine salts; and anionic surfactants such as sodium dodecylbenzenesulfonate, sodium alkyl sulfates, ammonium alkyl sulfates, sodium polyoxyethylene alkylether sulfates, ammonium polyoxyethylene alkylether sulfates, potassium salts of coconut oil fatty acid and cocoyl methyltaurine sodium salts.

The above-described aqueous emulsion is prepared, for example, by mixing a dimethylpolysiloxane and an emulsifier, adding water in portions while stirring, agitating the resulting mixture in a high shear mixer when a phase conversion occurs from W/O emulsion to O/W emulsion, adding a remaining portion of water, and agitating again in the high shear mixer.

The average particle size of the aqueous emulsion can be changed freely by changing the kind or amount of an emulsifier to be employed, or the stirring speed of the high shear mixer.

In the present invention, the average particle size of the emulsion is from 2 to 100 µm, being preferably from 3 to 50 µm, more preferably from 4 to 30 µm from the standpoint of its remaining property on the hair. The "average particle size" of the aqueous emulsion has the same meaning as that of the dimethylpolysiloxane as Component (C) in the aqueous hair cleansing agent.

The average particle size of a dimethylpolysiloxane emulsion can be measured using a particle size measuring instrument using a scattered laser light for example, "LS-130" (product of Coulter).

As an example of such dimethylpolysiloxane emulsion, a commercially available "Silicone CF2460" (product of Dow Corning Toray) obtained by mixing dimethylpolysiloxanes having a kinematic viscosity at 25°C of 8,000.000 mm²/s and 5,000 mm²/s at 1:1 and then emulsifying the resulting mixture with cetyltrimethylammonium chloride and having an average particle size of 20 µm can be used.

The content of the dimethylpolysiloxane as Component (C) is preferably from 0.01 to 10 weight% in the aqueous hair cleansing agent of the present invention. It is more preferably from 0.1 to 5 weight%, even more preferably from 0.5 to 2 weight% from the standpoints of smoothness during shampooing and rinsing and nonsticky feel after drying.

The aqueous hair cleansing agent of the present invention may contain a nonionic surfactant or amphoteric surfactant in order to improve the cleansing performance further.

Examples of the nonionic surfactant include polyoxyalkylene sorbitan fatty acid esters, polyoxyalkylene sorbitol fatty acid esters, polyoxyalkylene glycerol fatty acid esters, polyoxyalkylene fatty acid esters, polyoxyalkylene alkyl ethers, polyoxyalkylene alkyl phenyl ethers, polyoxyalkylene (hydrogenated) castor oils, sucrose fatty acid esters, polyglyceryl alkyl ethers, polyglyceryl fatty acid esters, fatty acid alkanolamides, alkyl glycosides, monoalkyl glyceryl ethers, and monoalkenyl glyceryl ethers.

Of these, polyoxyalkylene sorbitan fatty acid esters such as polyoxyethylene sorbitan fatty acid esters, polyoxyalkylene fatty acid esters such as polyoxyalkylene (C₈-C₂₀) fatty acid esters, polyoxyalkylene (hydrogenated) castor oils sucn as polyoxyethylene hydrogenated castor oil, and alkyl glycosides are preferred.

The fatty acid alkanolamides are also preferred. They may be either monoalkanolamides or dialkanolamides. They have preferably a C₈₋₁₈, more preferably C₁₀₋₁₆ acyl group. Those having a C₂₋₃ hydroxyalkyl group are also preferred. Examples include oleic acid diethanolamide, palm kernel oil fatty acid diethanolamide, coconut oil fatty acid diethanolamide, lauric acid diethanolamide, polyoxyethylene coconut oil fatty acid monoethanolamide, coconut oil fatty acid monoethanolamide, lauric acid isopropanolamide, and lauric acid monoethanolamide.

As Component (D), the monoalkyl glyceryl ethers and monoalkenyl glyceryl ethers are used. As the alkyl or alkenyl group of them, C₄₋₁₀ alkyl or alkenyl groups are preferred, with linear or branched C₈₋₁₀ alkyl or alkenyl groups being more preferred. Specific examples include n-butyl, isobutyl, n-pentyl, 2-methylbutyl, isopentyl, n-hexyl, isohexyl, n-heplyl, n-octyl, 2-ethylhexyl, n-decyl and isodecyl groups. Of these, 2-ethylhexyl and isodecyl groups are preferred.

Examples of the amphoteric surfactant include betaine surfactants. Of these, betaine surfactants such as alkyldimethylaminoacetic acid betaines, fatty acid amidopropylbetaines and alkylhydroxysulfobetaines are more preferred, of which fatty acid amidopropylbetaines are even more preferred. The fatty acid amidopropylbetaines have preferably a C₈₋₁₈ acyl group, more preferably a C₁₀₋₁₆ acyl group. Lauric acid amidopropylbetaine, palm kernel oil fatty acid amidopropylbetaine and coconut oil fatty acid amidopropylbetaine are even more preferred.

In the aqueous hair cleansing agent, these nonionic or amphoteric surfactants may be used either singly or in combination of two or more. In order to prepare a preferred aqueous hair cleansing agent of the present invention in the form of an aqueous liquid cleansing agent, use of a fatty acid amidopropylbetaine, fatty acid alkanolamide or monoalkyl glyceryl ether in combination with Component (A) is desired because combined use not only improves the foaming power but also gives the agent with adequate liquid properties.

The content of the nonionic or amphoteric surfactant in the aqueous hair cleansing agent of the present invention is preferably from 0.1 to 15 weight%, because it is effective for desirably increasing the foam volume. From this viewpoint, it is more preferably from 0.5 to 8 weight%, even more preferably from 1 to 6 weight%.

Furthermore, the aqueous hair cleansing agent of the present invention may contain a cationic surfactant, a cationized polymer other than Component (B) and a silicone other than Component (C) in order to improve the finish after drying.

Examples of the cationic surfactant include alkyltrimethylammonium salts, alkoxytrimethylammonium salts, dialkyldimethylammonium salts, alkyldimethylamines and salts thereof, alkoxydimethylamines and salts thereof, and alkylamidodimethylamines and salts thereof.
(i) As the alkyltrimethylammonium salts, those represented by the following formula can be used:

   R³-N⁺(CH₃)₃X⁻

   wherein, R³ represents a C₁₂-₂₂ alkyl group, and X⁻ represents a halide ion such as chloride ion or bromide ion. Specific examples include cetyltrimethylammonium chloride, stearyltrimethylammonium chloride and behenyltrimethylammonium chloride.
(ii) As the alkoxytrimethylammonium salts, those represented by the following formula can be used:

   R⁴-O-R⁵-N⁺ (CH₃)₃X⁻

   wherein, R⁴ represents a C₁₂₋₂₂ alkyl group, R⁵ represents an ethylene or propylene group which may be substituted with a hydroxy group, and X⁻ has the same meaning as described above.
   Specific examples include stearoxypropyltrimethylammonium chloride, stearoxyethyltrimethylammonium chloride and stearoxyhydroxypropyltrimethylammonium chloride.
(iii) As the dialkyldimethylammonium salts, those represented by the following formula can be used:

   (R⁶)₂N⁺(CH₃)₂X⁻

   wherein, R⁶ each independently represents a C₁₂₋₂₂ alkyl group or a benzyl group, and X⁻ has the same meaning as described above.
   Specific examples include distearyldimethylammonium chloride.
(iv) As the alkyldimethylamines and salts thereof, those represented by the following formula can be used:

   R⁷-N(CH₃)₂

   wherein, R⁷ represents a C₁₂₋₂₂ alkyl group.
   Specific examples include behenyldimethylamine and stearyldimethylamine, and organic acid salts thereof.
(v) As the alkoxydimethylamines and salts thereof, those represented by the following formula and salts thereof can be used:

   R⁸-O-R⁹-N(CH₃)₂

   wherein, R⁸ represents a C₁₂₋₂₂ alkyl group, and R⁹ represents an ethylene or propylene group.
(vi) As the alkylamidodimethylamines and salts thereof, those represented by the following formula and salts thereof can be used:

   R¹⁰-C(=O)NH-R¹¹-N(CH₃)₂

   wherein, R¹⁰ represents a C₁₁₋₂₁ alkyl group, and R¹¹ represents an ethylene or propylene group.

Examples of the cationic surfactant other than those described in (i) to (vi) include lanolin fatty acid aminopropylethyldimethylammonium ethyl sulfate, lanolin fatty acid aminoethyltriethylammonium ethyl sulfate, lanolin fatty acid aminopropyltriethylammonium ethyl sulfate, lanolin fatty acid aminoethyltrimethylammonium methyl sulfate, lanolin fatty acid aminopropylethyldimethylammonium methyl sulfate, isoalkanoic acid (C₁₄-C₂₀) aminopropylethyldimethylammonium ethyl sulfate, isoalkanoic acid (C₁₈-C₂₂) aminopropylethyldimethylammonium ethyl sulfate, isostearic acid aminopropylethyldimethylammonium ethyl sulfate, isononanoic acid aminopropylethyldimethylammonium ethyl sulfate, and alkyltrimethylammonium saccharins.

These cationic surfactants may be used in combination of two or more. The content thereof in the aqueous hair cleansing agent of the present invention is preferably from 0.01 to 10 weight%, more preferably from 0.05 to 6 weight%, even more preferably from 0.3 to 3 weight%, even more preferably from 0.5 to 2 weight% from the viewpoint of smoothness during shampooing and rinsing.

Examples of the cationized polymer other than Component (B) include cationized cellulose, cationized starch, cationized guar gum, cationized locust bean gum, diallyl dialkyl ammonium salt/acrylamide copolymers, vinylimidazolium chloride/vinylpyrrolidone copolymers, hydroxyethyl cellulose/dimethyldiallylammonium chloride copolymers, vinylpyrrolidone/quaternized dimethylaminoethyl methacrylate copolymers, vinylpyrrolidone/alkylamino acrylate copolymers, vinylpyrrolidone/alkylamino acrylate/vinylcaprolactam copolymers, vinylpyrrolidone/methacrylamidopropyl trimethylammonium chloride copolymers, alkylacrylamide/alkyl acrylate/alkylaminoalkylacrylamide/polyethylene glycol methacrylate copolymers, adipic acid/dimethylaminohydroxypropylethylenetriamine copolymer ("Cartaretin", product of Sandoz/USA), and cationic polymers as described in JP-A-53-139734 and JP-A-60-36407. Of these, cationized cellulose, cationized guar gum, and diallyldialkyl ammonoium salt/acrylamide copolymers are preferred.

In addition, commercially available products such as "Merquat 550" (a copolymer of acrylamide and diallyldimethylammonium salt; CTFA name: Polyquaternium-7; product of ONDEO-NALCO), "Luviquat FC370" (a copolymer of 1-vinyl-2-pyrrolidone and 1-vinyl-3-methylimidazolium salt; CTFA name: polyquaternium-16; product of BASF), "Gafquat 755N" (a copolymer of 1-vinyl-2-pyrrolidone and dimethylaminoethyl methacrylate; CTFA name: polyquaternium-11; product of ISP), "Ucare Polymer JR series" and "Polymer LR series" (salt of a reaction product of trimethylammonium substituted epoxide with hydroxyethyl cellulose; CTFA name: polyquaternium-10; product of Amerchol), "Poiz C-60H", "Poiz C-80M" and "Poiz-C150L" (salts of a reaction product of trimethylammonium substituted epoxide with hydroxyethyl cellulose; CTFA name; polyquaternium-10; product of Kao); "Jaguar series" (guar hydroxypropyltrimethylammonium chloride; product of Rhodia); and "Catinal CLB-100" (locust bean hydroxypropyltrimonium chloride, product of Toho Chemical Industry).

These cationized polymers other than Component (B) may be used in combination of two or more. From the viewpoint of the smoothness during shampooing and rinsing, its content in the aqueous hair cleansing agent of the present invention preferably ranges from 0.01 to 3 weight%, more preferably from 0.05 to 2 weight%, even more preferably from 0.1 to 0.5 weight%.

The below-described silicones, for example, can be used as those other than Component (C).

### (1) Amino-modified silicones

Various amino-modified silicones can be used. Those having an average molecular weight of from about 3000 to 100,000 and listed under the name of "Amodimethicone" in the third edition of CTFA Dictionary (Cosmetic Ingredient Dictionary/USA) are preferred. Commercially available products include "SM 8704C" (trade name; product of Dow Corning Toray), "DC 929" (trade name; product of Dow Corning), "KT1989" (trade name; product of GE Toshiba Silicones), "8500 Conditioning Agent", "DOW CORNING TORAY SS-3588" and "DOW CORNING TORAY SILSTYLE 104" (each, product of Dow Corning Toray).

### (2) Other silicones

Examples of the silicone other than those described above include polyether modified silicones, methylphenylpolysiloxane, fatty acid modified silicones, alcohol modified silicones, alkoxy modified silicones, epoxy modified silicones, fluorine modified silicones, cyclic silicones and alkyl modified silicones.

Two or more of these silicones may be used in combination. The content thereof in the aqueous hair cleansing agent of the present invention is preferably from 0.01 to 10 weight%, more preferably from 0.05 to 5 weight%, even more preferably from 0.1 to 2 weight% from the viewpoint of the smoothness during shampooing and rinsing.

The aqueous hair cleansing agent of the present invention may contain, as a pearling agent, an ethylene glycol monofatty acid ester, ethylene glycol difatty acid ester, ethylene glycol monoalkyl ether or ethylene glycol dialkyl ether.

Examples of the ethylene glycol monofatty acid ester include ethylene glycol monostearate and ethylene glycol monobehenate; and those of the ethylene glycol difatty acid ester include ethylene glycol distearate and ethylene glycol dibehenate. Examples of the ethylene glycol monoalkyl ether include ethylene glycol monostearyl ether, while those of the ethylene glycol dialkyl ether include ethylene glycol distearyl ether.

Two or more of these pearling agents may be used in combination and the content thereof in the aqueous hair cleansing agent of the present invention is preferably from 0.1 to 10 weight%, more preferably from 0.5 to 5 weight%, even more preferably from 1 to 4 weight% from the standpoints of improving the storage stability of the aqueous hair cleansing agent and improving its smoothness during foaming and rinsing.

The aqueous hair cleansing agent of the present invention may contain an oil as another conditioning agent. Examples of the oil include hydrocarbons such as squalene, squalane, liquid paraffin, liquid isoparaffin, and cycloparaffin; oils or fats such as castor oil, cacao oil, mink oil, avocado oil, olive oil, sunflower oil and camellia oil; waxes such as beeswax, whale wax, lanolin, and carnauba wax; higher alcohols such as cetyl alcohol, oleyl alcohol, stearyl alcohol, isostearyl alcohol, 2-octyldodecanol, myristyl alcohol, behenyl alcohol and cetostearyl alcohol; esters such as isopropyl palmitate, isopropyl myristate, octyldodecyl myristate, hexyl laurate, cetyl lactate, propylene glycol monostearate, oleyl oleate, hexadecyl 2-ethylhexanoate, isononyl isononanoate and tridecyl isononanoate; higher fatty acids such as capric acid, lauric acid, myristic acid, palmitic acid, stearic acid, behenic acid, oleic acid, coconut oil fatty acids, isostearic acid, and isopalmitic acid; and other oils such as isostearyl glyceryl ether and polyoxypropylene butyl ether. Of these, higher alcohols and oils or fats are preferred, with myristyl alcohol, cetyl alcohol, stearyl alcohol, sunflower oil and camellia oil are more preferred. These oils may be use either singly or in combination of two or more. The content thereof in the aqueous hair cleansing agent of the present invention is preferably from 0.1 to 2 weight%, more preferably from 0.2 to 1.5 weight%, even more preferably from 0.3 to 1 weight%.

The aqueous hair cleansing agent of the present invention may contain a viscosity regulator. Examples of the viscosity regulator include hydroxyethyl cellulose, methyl cellulose, polyethylene glycol, polypropylene glycol, ethylene glycol, propylene glycol, isoprene glycol, ethanol, benzyl alcohol, benzyl oxyethanol, phenoxyethanol, clay minerals, and salts such as sodium chloride, ammonium chloride, sodium citrate and the like. Of these, benzyl alcohol, ethanol, polypropylene glycol, sodium chloride and sodium citrate are preferred. Two or more of these viscosity regulators may be used in combination. The amount used thereof in the aqueous hair cleansing agent of the present invention is preferably from 0.01 to 5 weight%, more preferably from 0.05 to 4 weight%, even more preferably from 0.1 to 3 weight% from the standpoints of the volume and quality of foams.

In addition to the above-described components, components which are employed in ordinary aqueous hair cleansing agents can be incorporated in the aqueous hair cleansing agent of the present invention as needed depending on the purpose of use. Such components include, for example, antidandruff agents; vitamin preparations; bactericides; anti-inflammatories; antiseptics; chelating agents; humectants such as glycerin, sorbitol and panthenol; colorants such as dyes and pigments; extracts such as extracts of eucalyptus in a polar solvent, proteins available from shells having a pearl layer or pearls or hydrolysates thereof, honey, royal jelly, proteins available from silk or hydrolysates thereof, protein-containing extracts available from seeds of leguminous plants, Asian ginseng extract, rice bran extract, *Fucus vesiculosus* extract, aloe extract, Alpinia Leaf extract, and chlorella extract; pearling agents such as titanium oxide; perfumes; ultraviolet absorbers; antioxidants; and other components listed in ENCYCLOPEDIA OF SHAMPOO INGREDIENTS (MICELLE PRESS).

The aqueous hair cleansing agent of the present invention has preferably a pH (25°C, diluted 20-fold with water) of from 2 to 6, more preferably from 3 to 5, even more preferably from 3.5 to 4.5 when it is applied to the hair, from the viewpoint of improving the luster and manageability of the hair. As a pH regulator, organic acids are preferred, with α-hydroxy acids being more preferred. Specific preferred examples include malic acid, citric acid, lactic acid and glycolic acid. As the pH regulator, two or more of these organic acids may be used in combination. The amount used thereof is'preferably from 0.01 to 5 weight%, more preferably from 0.1 to 3 weight%, even more preferably from 0.3 to 2 weight% in the aqueous hair cleansing agent of the present invention from the viewpoints of improvement in foam quality and flexibility of hair during shampooing. As another pH regulator, a base such as sodium hydroxide, potassium hydroxide or ammonium chloride may be used in combination with the above-described organic acid.

Although the form of the aqueous hair cleansing agent of the present invention can be chosen as desired from a liquid form, a gel form and the like, a liquid form in a solvent such as water or a lower alcohol is preferred, with the former one being more preferred.

### -Examples-

### Preparation Example 1: Preparation of a dimethylpolysiloxane emulsion 1 (having an effective content of 60 weight% and an average particle size of 2 µm)

20 parts by weight of a dimethylpolysiloxane having a kinematic viscosity at 25°C of 6,000,000 mm²/s and 40 parts by weight of a dimethylpolysiloxane having a kinematic viscosity at 25°C of 500 mm²/s were mixed using "Three-One Motor BL600" (product of HEIDON) at a rotation speed of 200 rpm into a homogeneous mixture. While stirring the resulting mixture at a rotation speed of 200 rpm, 3.5 parts by weight of polyoxyethylene (23) lauryl ether and 1.5 parts by weight of polyoxyethylene (4) lauryl ether were added thereto and they were mixed for 10 minutes. While stirring at a rotation speed of 200 rpm, 25 parts by weight of water was added, followed by stirring for 5 minutes at a rotation speed of 5,000 rpm by using "AGI HOMOMIXER" (f2/5 model, No. 023010) of Tokushu Kika Kogyo. To the resulting mixture was added 10 parts by weight of water and they were mixed for 5 minutes at a rotation speed of 5000 rpm by using "AGI HOMOMIXER" mentioned above.

An average particle size of the emulsified particles in the silicon emulsion was measured by laser diffraction measurement at an appropriate concentration by using "LS-130", product of Coulter, resulting in an average particle size of 2.0 µm.

### Preparation Example 2: Preparation of a dimethylpolysiloxane emulsion 2 (having an effective content of 60 weight% and an average particie size of 4 µm)

In a similar manner to that employed in Preparation Example 1 except that 55 parts by weight of a dimethylpolysiloxane having a kinematic viscosity of 10,000 mm²/s was mixed with 5 parts by weight of a dimethylpolysiloxane of 10 mm²/s and the rotation speed of AGI HOMOMIXER was changed to 3000 rpm, a dimethylpolysiloxane emulsion was prepared.

The average particle size of the emulsified particles was measured as in Preparation Example 1, resulting in that of 4.0 µm.

### Examples 1 and 2 and Comparative Examples 1 to 4 (Examples 1 and 2 are Reference Examples)

Hair cleansing agents shown in Table 1 were prepared using the dimethylpolysiloxane emulsions obtained in Preparation Examples 1 and 2, respectively, and they were evaluated by the below-described evaluation method. The results are also shown in Table 1. The pH is measured at 25°C after the agent is diluted 20-fold with water.

### (1) Foaming speed

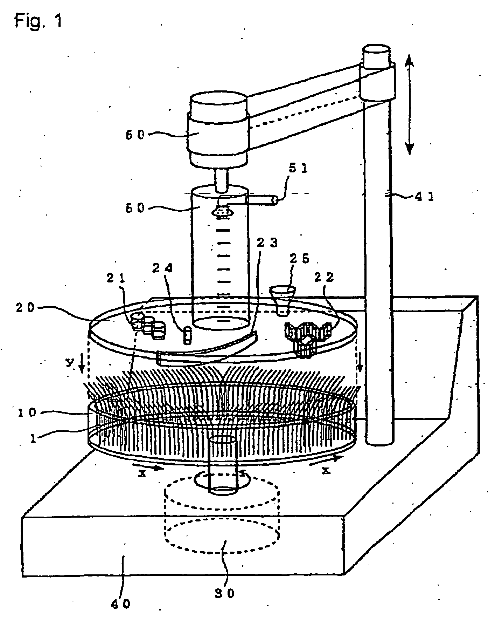

The foaming speed was measured using an apparatus shown in FIG. 1.

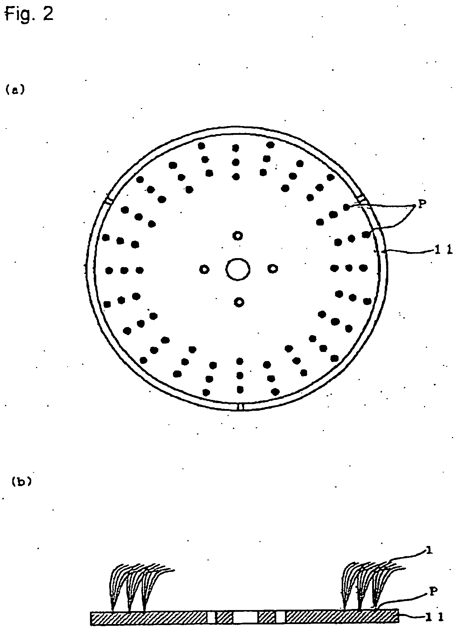

The apparatus of FIG. 1 is equipped with an open-topped cylindrical container 10 (diameter: 160 mm, height: 55 mm) made of transparent plastic and having a drain outlet (not illustrated) at the bottom of the container, a lid 20 made of transparent plastic, a control unit 40 having a motor 30 loaded therein, a metering instrument 50 and a torque detector 60. A disk 11 (diameter: about 160 mm) having a plurality of implant holes P into which hairs 1 (90 mm, 30 g in total) have been implanted as illustrated in FIG. 2 is installed inside the container 10. The container 10 is rotated in the direction of an arrow xby the motor 30, whereby the hairs 1 in the container 10 are also rotated.

The lid 20 has, on the back side thereof, first protrusions 21 (3 cylindrical protrusions each having a diameter of 15 mm and height of 12 mm) corresponding to human fingers and second protrusions 22 (9 protrusions each having length of 10 mm, width of 2 mm and height of 12 mm) corresponding to a brush. The lid 20 is brought into contact with the hairs 1 when the lid 20 is put on the upper surface of the container 10 in the direction of an arrow y. The lid 20 has a funnel-like inlet 25 for pouring a cleansing agent therefrom. When the lid 20 is put on the container 10, it is not fixed to the container 10 but is supported by a support 41 via the torque detector 60 and metering instrument 50. Accordingly, rotation of the container 10 with the lid 20 thereon causes a sliding contact of the hairs 1 in the container 10 with the protrusions 21 and 22. When the cleansing agent is poured onto the hairs 1 from the inlet 25, the cleansing agent can therefore be foamed as if the hairs are actually shampooed.

First, the hairs were wetted with 30 g of water and 0.3 mL of a model sebum made of lanolin was added. Then, 1.5 mL of a sample to be evaluated was poured. The container 10 was then rotated at a speed of 70 revolutions per minute to cause a sliding contact between the hairs 1 and the protrusions 21 and 22. Foams thus generated were collected in the metering instrument 50 and the cleansing agent was evaluated based on the time until the foam volume reached 25 mL in accordance with the below-described criteria.
A: less than 100 seconds
B: 100 seconds or greater but less than 200 seconds
C: 200 seconds or greater but less than 300 seconds
D: 300 seconds or greater

### (2) Smoothness during foaming

After a human hair tress of 25 cm in length, 5.5 cm in width and 10 g in weight was rinsed lightly with hot water of 40°C, excess water was removed. 0.5 g of a hair cleansing agent was applied thereon to foam sufficiently for about 30 seconds. The smoothness was organoleptically evaluated during the foaming. Evaluation was carried out by a panel of five experts and their total scores were indicated. Criteria for evaluation are as follows:
4: Very smooth
3: Smooth
2: Not so smooth
1: Not smooth

### (3) Smoothness during rinsing

After a human hair tress of 25 cm in length, 5.5 cm in width and 10 g in weight was rinsed lightly with hot water of 40°C, excess water was removed. 0.5 g of a hair cleansing agent was applied thereon to foam sufficiently for about 30 seconds. The smoothness was organoleptically evaluated while rinsing the lathered hair tress with hot water of 40°C having a flow rate of 2 L/min. Evaluation was carried out by a panel of five experts and their total scores were indicated. Criteria for evaluation are as follows:
4: Very smooth
3: Smooth
2: Not so smooth
1: Not smooth

### (4) Luster and manageability after drying

A hair tress treated in a similar manner as the evaluation of smoothness was rinsed with running water (2 L/min) of 40°C and then towel-dried sufficiently. After natural drying, the luster and manageability were evaluated visually. They were evaluated by a panel of 5 experts and total scores were indicated. Criteria for evaluation are as described below:
4: Very good
3: Good
2: Not so good
1: Not good

### (5) Stability

The hair cleansing agent (100 mL) was filled in a 110-mL capped bottle and after storage at 50°C for 1 month, it was evaluated in accordance with the following evaluation criteria.
A: No change is observed after storage.
B: A slight change is observed after storage (within an allowance)
C: An apparent change is observed after storage (outside an allowance)

**Table 1**

| (weight%) | | Examples | | Comparative Examples | | | |
|---|---|---|---|---|---|---|---|
| | | 1 | 2 | 1 | 2 | 3 | 4 |
| Component (A) | Ammonium lauryl ether (1) sulfate *1 | 12 | 12 | 12 | 12 | 12 | 12 |
| Component (B) | Cationized tara gum*² | 0.5 | 0.5 | 0.5 | - | - | - |
| Comparative Component (B') | Cationized hydroxyethyl cellulose *3 | - | - | - | 0.5 | - | - |
| | CationQed guar gum*⁴ | - | - | - | - | 0.5 | 0.5 |
| Component (C) | Dimethylpolysiloxane emulsion (Preparation Example 1) | 1.7 | - | - | 1.7 | - | - |
| | Dimethylpolysiloxane emulsion (Preparation Example 2) | - | 1.7 | - | - | 1.7 | - |
| Comparison Component (C') | Dimethylpolysiloxane emulsion *5 | - | - | 1.7 | - | - | 1.7 |
| | Ethylene glycol distearate | 2 | 2 | 2 | 2 | 2 | 2 |
| | Lauroytamidopropylbetaine | 1.5 | 1.5 | 1.5 | 1.5 | 1.5 | 1.5 |
| Other components | Myristyl alcohol | 1 | 1 | 1 | 1 | 1 | 1 |
| | Cocoyl monoethanolamide | 0.5 | 0.5 | 0.5 | 0.5 | 0.5 | 0.5 |
| | Benzyl alcohol | 0.5 | 0.6 | 0.5 | 0.5 | 0.5 | 0.5 |
| | Sodium chloride | 0.2 | 0.2 | 0.2 | 0.2 | 0.2 | 0.2 |
| | Malic acid | 0.7 | 0.7 | 0.7 | 0.7 | 0.7 | 0.7 |
| | Purified water | Balance | Balance | Balance | Balance | Balance | Balance |
| Total | | 100 | 100 | 100 | 100 | 100 | 100 |
| pH | | 3.9 | 3.9 | 3.9 | 3.9 | 3.9 | 3.9 |
| | Foaming speed | A | A | B | B | C | B |
| Evaluation | Smoothness during foaming | 19 | 20 | 12 | 13 | 14 | 11 |
| | Smoothness during rinsing | 18 | 20 | 14 | 10 | 15 | 12 |
| | Luster and manageability after drying | 19 | 18 | 14 | 15 | 11 | 13 |
| | Stability | A | A | A | C | B | A |
| *1: Ammonium polyoxyethylene (1) lauryl ether sulfate; average number of moles of ethylene oxide: 1 | | | | | | | |
| *2: Cationized tara gum: "Catinal CTR-100" (product of Toho Chemical Industry) | | | | | | | |
| *3: Cationized hydroxyethyl cellulose; "Poiz C-80M" (product of Kao) | | | | | | | |
| *4: Cationized guar gum; "Jaquar C-13S" (product of Rhodia) | | | | | | | |
| *5: Dimethylpolysiloxane emulsion: commercially available as "Silicone CF2450" from Dow Corning Toray containing 25 weight% of a dimethylpolysiloxane having a kinematic viscosity of 6,000,000 mm²/s and 35 weight% of a dimethylpolysiloxane having a kinematic viscosity of 500 mm²/s, respectively, and having an average particle size of 0.7 µm. | | | | | | | |

### Example 3: Conditioning shampoo

| | (weight%) |
|---|---|
| Ammonium polyoxyethylene (1) lauryl ether sulfate (average number of moles of ethylene oxide: 1) | 13.0 |
| Cationized tara gum ("Catinal "CTR-100", product of Toho Chemical Industry) | 0.3 |
| Dimethylpolysiloxane emulsion (Dimethylpolysiloxane Emulsion 2 of Preparation Example 2) | 1.7 |
| Isodecyl glyceryl ether (derived from alcohol obtained by the conversion of a trimer of propylene by the oxo process, which will equally apply hereinafter) | 0.5 |
| Amino-modified silicone ("8500 Conditioning Agent", Product of Dow Corning Toray) | 0.3 |
| Cationized cellulose ("Poiz C-80M", product of Kao) | 0.3 |
| Diallyldimethylammonium chloride/acrylamide copolymer ("Merquat 550", product of Ondeo Nalco, effective content: 8.5 weight%) | 1.7 |
| Ethylene glycol distearate | 2.0 |
| Lauroyl amidopropylbetaine | 1.0 |
| Myristyl alcohol | 0.4 |
| Cetyl alcohol | 0.1 |
| Cocoyl monoethanolamide | 0.5 |
| Polyoxyethylene (16) lauryl ether | 0.7 |
| Polypropylene glycol (weight average molecular weight: 400) | 0.5 |
| Benzyl alcohol | 0.3 |
| Ethanol | 3.0 |
| Camellia oil | 0.01 |
| Panthenol | 0.05 |
| Royal jelly | 0.01 |
| Purified honey | 0.01 |
| Silk extract | 0.05 |
| Sodium chloride | 0.2 |
| Perfume | q.s. |
| Malic acid | 0.5 |
| pH regulator (lactic acid) | An amount to adjust pH to 3.9 |
| Ion exchange water | Balance |

### Example 4 Conditioning shampoo

| | (weight%) |
|---|---|
| Sodium polyoxyethylene (2) lauryl ether sulfate (average number of moles of ethylene oxide: 2) | 13.0 |
| Cationized tara gum ("Catinal CTR-200", product of Toho Chemical Industry) | 0.4 |
| Dimethylpolysiloxane emulsion ("Silicone CF2960", product of Dow Corning Toray, average particle size: 20 µm, effective content: 75 weight%) | 3.0 |
| Isodecyl glyceryl ether | 0.7 |
| Amino-modified silicone ("SS-3588", product of Dow Corning Toray) | 0.1 |
| Cationized guar gum ("Jaguar C-17", product of Rhodia) | 0.1 |
| Diallyldimethylammonium chloride homopolymer ("Merquat 100", product of Ondeo Nalco, Effective content: 40 weight%) | 0.8 |
| Ethylene glycol distearate | 3.0 |
| Lauroyl amidopropylbetaine | 2.0 |
| Cocoyl monoethanolamide | 0.5 |
| Myristyl alcohol | 0.5 |
| Cetyl alcohol | 0.5 |
| Polyoxyethylene (16) lauryl ether | 1.0 |
| Benzyl alcohol | 1.0 |
| Propylene glycol (weight average molecular weight: 400) | 0.2 |
| Sodium chloride | 1.0 |
| Hydrolyzed conchiolin solution (dry content: 3 weight%) | 0.05 |
| Asian ginseng extract (dry content: 3 weight%) | 0.05 |
| Soybean extract (dry content: 0.4 weight%) | 0.05 |
| Eucalyptus extract (dry content: 0.2 weight%) | 0.05 |
| Rice bran oil | 0.05 |
| Glycolic acid | 1.0 |
| Perfume | q.s. |
| pH regulator (sodium hydroxide) | Amount to adjust pH to 3.9 |
| Ion exchange water | Balance |

### Example 5 Conditioning shampoo

| | (weight%) |
|---|---|
| Sodium polyoxyethylene (1) lauryl ether sulfate (average number of moles of ethylene oxide) | 16.0 |
| Cationized tara gum ("Catinal "CTR-100", product of Toho Chemical Industry) | 0.8 |
| Dimethylpolysiloxane emulsion (Dimethylpolysiloxane emulsion 1 of Prep. Ex. 1) | 3.0 |
| 2-Ethylhexyl glyceryl ether | 0.3 |
| Amino-modified silicone ("8500 Conditioning agent", Product of Dow Corning Toray) | 0.5 |
| Ethylene glycol distearate | 3.0 |
| Cocoyl monoethanolamide | 0.5 |
| Stearyl alcohol | 1.0 |
| Glycerin | 1.0 |
| Sodium chloride | 0.2 |
| Benzyloxyethanol | 0.5 |
| Malic acid | 0.7 |
| Perfume | q.s. |
| Lactic acid | 0.1 |
| pH regulator (citric acid) | Amount to adjust pH to 5.5 |
| Ion exchange water | Balance |

### Example 6 Conditioning shampoo

| | (weight%) |
|---|---|
| Sodium polyoxyethylene (2.5) lauryl ether sulfate (average number of moles of ethylene oxide: 2.5) | 15.0 |
| Cationized tara gum ("Catinal CTR-200", product of Toho Chemical Industry) | 0.2 |
| Dimethylpolysiloxane emulsion (Dimethylpolysiloxane emulsion 2 of Prep. Ex. 2) | 2.0 |
| 2-Ethylhexyl glyceryl ether | 1.5 |
| Amino-modified silicone ("SILSTYLE 104", product of Dow Corning Toray) | 1.0 |
| Cationized locust bean gum ("Catinal CLB-100", Product of Toho Chemical Industry) | 0.2 |
| Ethylene glycol distearate | 2.0 |
| Lauroyl amidopropylbetaine | 3.0 |
| Cocoyl monoethanolamide | 0.8 |
| Polyoxyethylene (16) lauryl ether | 2.0 |
| Sodium Cocoamphoacetate | 1.0 |
| Stearoxypropyl dimethylamine-malate salt | 0.5 |
| Polypropylene glycol (weight average molecular weight: 400) | 0.5 |
| Sodium chloride | 1.0 |
| Malic acid | 0.8 |
| Citric acid | 0.75 |
| pH regulator (sodium hydroxide) | Amount to adjust pH to 3.5 |
| Ion exchange water | Balance |

Hair cleansing agents obtained in Examples 3 to 6 lathered quickly, were excellent in the smoothness of the hair during foaming and rinsing, provided excellent luster and manageability to the finished hair, and had excellent storage stability.

## Claims

1. An aqueous hair cleansing agent comprising the following components (A), (B), (C) and (D):
(A) an anionic surfactant;
(B) a cationized tara gum;
(C) a dimethylpolysiloxane present as dispersed particles having an average particle size of from 2 to 100 µm; and
(D) a glyceryl ether selected from the group consisting of monoalkyl glyceryl ethers having a C₄₋₁₀ alkyl group and monoalkenyl glyceryl ethers having a C₄₋₁₀ alkenyl group.

2. The aqueous hair cleansing agent according to Claim 1, comprising from 1 to 30 weight% of Component (A), from 0.01 to 10 weight% of Component (B) and from 0.01 to 10 weight% of Component (C).

3. The aqueous hair cleansing agent according to Claim 1 or 2, wherein Component (A) is represented by the following formula (1) or (2):
R¹O (CH₂CH₂O)ₘSO₃M (1)
R¹OSO₃M (2)
wherein, R¹ represents a C₁₀₋₁₈ alkyl or alkenyl group, M represents an alkali metal, alkaline earth metal, ammonium, or a cation derived from alkanolamine or basic amino acid, and m stands for a number from 1 to 5 on weight average.

4. The aqueous hair cleansing agent according to any one of Claims 1 to 3, which has a pH of from 2 to 6 at 25°C when diluted 20-fold with water.

## Patentansprüche

1. Wässriges Haarreinigungsmittel, das die folgenden Komponenten (A), (B), (C) und (D) umfasst:
(A) ein anionisches Tensid;
(B) einen kationisierten Taragummi;
(C) Dimethylpolysiloxan, das in Form dispergierter Partikel mit einer mittleren Partikelgröße von 2 bis 100 µm vorliegt; und
(D) einen Glycerylether, ausgewählt aus der Gruppe, bestehend aus Monoalkylglycerylethern mit einer C₄₋₁₀-Alkylgruppe und Monoalkenylglycerylethern mit einer C₄₋₁₀-Alkenylgruppe.

2. Wässriges Haarreinigungsmittel gemäß Anspruch 1, das 1 bis 30 Gew.% der Komponente (A), 0,01 bis 10 Gew.% der Komponente (B) und 0,01 bis 10 Gew.% der Komponente (C) umfasst.

3. Wässriges Haarreinigungsmittel gemäß Anspruch 1 oder 2, worin Komponente (A) durch die folgende Formel (1) oder (2) dargestellt wird:
R¹O(CH₂CH₂O)ₘSO₃M (1)
R¹OSO₃M (2)
worin R¹ eine C₁₀-₁₈-Alkyl- oder -Alkenylgruppe darstellt, M ein Alkalimetall, Erdalkalimetall, Ammonium oder ein von Alkanolamin oder einer basischen Aminosäure abgeleitetes Kation darstellt, und m für eine Zahl von 1 bis 5 im Gewichtsmittel steht.

4. Wässriges Haarreinigungsmittel gemäß irgendeinem der Ansprüche 1 bis 3, das einen pH-Wert von 2 bis 6 bei 25°C hat, wenn es 20-fach mit Wasser verdünnt ist.

## Revendications

1. Agent aqueux de nettoyage de cheveux comprenant les composants (A), (B) (C) et (D) :
(A) un tensioactif anionique ;
(B) une gomme tara cationisée ;
(C) un diméthylpolysiloxane présent sous forme de particules dispersées ayant une taille de particule moyenne allant de 2 à 100 µm ; et
(D) un éther de glycéryle choisi dans le groupe constitué d'éthers de glycéryle monoalkyliques ayant un groupe alkyle en C₄-C₁₀ et d'éthers de glycéryle monoalcényliques ayant un groupe alcényle en C₄-C₁₀.

2. Agent aqueux de nettoyage de cheveux selon la revendication 1, comprenant de 1 à 30% en poids d'un composant (A), de 0,01 à 10% en poids d'un composant (B) et de 0,01 à 10% en poids d'un composant (C).

3. Agent aqueux de nettoyage de cheveux selon la revendication 1 ou 2, dans lequel le composant (A) est représenté par la formule (1) ou (2) suivante :
R¹O(CH₂CH₂O)ₘSO₃M (1)
R¹OSO₃M (2)
où, R¹ représente un groupe alkyle ou alcényle en C₁₀-C₁₈, M représente un métal alcalin, un métal alcalino-terreux, un ammonium, ou un cation dérivé d'une alkanolamine ou d'un acide aminé basique, et m désigne un nombre allant de 1 à 5 en moyenne en poids.

4. Agent aqueux de nettoyage de cheveux selon l'une quelconque des revendications 1 à 3, qui a un pH allant de 2 à 6 à 25°C lorsqu'il est dilué 20 fois avec de l'eau.
